# EUROPEAN PATENT APPLICATION

(11) **EP 1 120 123 A1**
(43) Date of publication of application: **01.08.2001**
(21) Application number: 01101927.0
(22) Date of filing: 29.01.2001
(51) Int. Cl.: A61L 33/00, A61L 33/10

(54) **Antithrombogenic composition and medical device coated with the same**

(30) Priority: 27.01.2000 JP 2000018194
(71) Applicant: Toyo Boseki Kabushiki Kaisha, Osaka-shi, Osaka 530-8230 (JP)
(72) Inventor: Kashiwabara, Susumu, c/oToyo Boseki K.K Resea. Ce., Ohtsu-shi, Shiga 502-0292 (JP); Tanaka, Hidenori, c/oToyo Boseki K.K Research Cent, Ohtsu-shi, Shiga 502-0292 (JP); Sato, Masaki, c/oToyo Boseki K.K Research Cent, Ohtsu-shi, Shiga 502-0292 (JP)
(74) Representative: Weber, Thomas, Dr. Dipl.-Chem.

(57) **Abstract**

The present invention provides an antithrombogenic composition containing an ionic complex of ammonium salts and heparin or a heparin derivative, the ammonium salts each comprising four aliphatic alkyl groups bonded thereto, wherein an ammonium salt containing four aliphatic alkyl groups having not less than 22 and not more than 26 carbon atoms in total is contained in an amount of not less than 5% and not more than 80% of the total ammonium salt by weight, which is capable of maintaining sufficient antithrombogenicity for a long time, and a medical device coated with the antithrombogenic composition.

## Description

### TECHNICAL FIELD OF THE INVENTION

The present invention relates to an antithrombogenic composition particularly useful as a coating material used for a medical device that comes into direct contact with blood. More particularly, the present invention relates to a medical device produced by applying the antithrombogenic composition to a conventional medical device, which has superior antithrombogenicity and which is free from degradation of the function a medical device inherently has.

### BACKGROUND OF THE INVENTION

Many attempts have been made to develop and practice a technology for imparting antithrombogenicity to a medical device that comes into direct contact with blood, so that the blood would not coagulate on the surface of the device. Examples of the device include circuit tubes for blood, tubes for sampling monitor, intraaortic balloon pumps, blood pumps for artificial heart, angiography catheters, artificial lung, venous reservoir, artificial kidney, artery filters and the like. The antithrombogenicity is mainly achieved by immobilizing heparin having an anticoagulant effect or a derivative thereof on the surface of a medical device by some method. Heparin is known to form a complex with antithrombin III in plasma and show a strong anticoagulant effect such as an antithrombin effect, an anti-factor Xa effect and the like.

Japanese Publication for Opposition No. 48-13341 discloses a method comprising reacting heparin with a cationic surfactant to give a heparin complex insoluble in water and soluble in an organic solvent, which is then dissolved in an organic solvent solely or with plastics, applying the resulting solution to the surface of plastic and drying the surface to form an antithrombogenic surface. In this publication, examples of the surfactant are, alkyltrimethylammonium chloride, dilauryldimethylammonium chloride and the like.

However, ionic complexes of these surfactants and heparin easily dissolve in blood, and sufficient antithrombogenicity cannot be provided over a long period of time.

To solve this problem, a complex of heparin and a benzyldimethylstearylammonium salt, which is a benzalkonium salt (ammonium salt having one long chain alkyl group and two methyl groups, and one benzyl group) containing an alkyl group having 18 carbon atoms in total has been disclosed. This complex prevents early disappearance of activity of a conventional complex consisting of a cationic surfactant and heparin. However, this complex (used in Comparative example) fails to overcome the defect of losing the antithrombogenicity in a relatively short time.

J. Biomater. Sci. Polym. Ed., vol. 6 describes a complex of heparin and dioctadecylammonium bromide (used in Comparative example). This compound remains on the surface of the material over a long time due to the high hydrophobicity of the cationic compound bonded thereto. However, the hydrophobicity of the bonded cationic group is too high, which greatly suppresses the activity of heparin. When it is used as a coating agent of a medical device, therefore, thrombus tends to be formed in the blood if it remains at the same place like a reservoir, in the blood pooled in an uneven part on the surface and the like of the medical device, which poses a problem.

The problem of a complex of heparin and an organic cationic group is that, when an organic cationic group (e.g., benzalkonium salt) having high hydrophilicity is used, the activity cannot be maintained for a long time due to the early dissolution, and, when an organic cationic group having high hydrophobicity is used, the heparin activity becomes low and sufficient antithrombogenicity cannot be exerted.

### SUMMARY OF THE INVENTION

According to the present invention, the above-mentioned defects can be solved based on the finding that the use of only one kind of ammonium salt when forming a complex with heparin has caused the above-mentioned defects. In other words, inasmuch as only one kind of ammonium salt is bonded to the anionic group of heparin, the property of the resulting complex varies depending on the total number of carbon atoms of the alkyl chain of the ammonium salt. As a result, the complex becomes too hydrophilic or too hydrophobic, which in turn causes either dissolution of the complex at an early stage or great suppression of the dissolution. In either case, the expected efficacy cannot be offered. It has been found according to the present invention that a complex of plural kinds of ammonium salts having different total number of carbon atoms of alkyl groups, and heparin or a heparin derivative is effective for providing sufficient antithrombogenicity necessary for a medical device that comes into direct contact with blood and for the maintenance thereof over a long period of time.

Thus, the present invention provides the following.
(1) An antithrombogenic composition comprising an ionic complex of ammonium salts and heparin or a heparin derivative, said ammonium salts each comprising four aliphatic alkyl groups bonded thereto, wherein an ammonium salt comprising four aliphatic alkyl groups having not less than 22 and not more than 26 carbon atoms in total is contained in an amount of not less than 5% and not more than 80% of the total ammonium salt by weight.
(2) The antithrombogenic composition of the above-mentioned (1), wherein an ammonium salt other than the ammonium salt comprising four aliphatic alkyl groups having not less than 22 and not more than 26 carbon atoms in total comprises four aliphatic alkyl groups having not less than 27 carbon atoms in total.
(3) The antithrombogenic composition of the above-mentioned (2), wherein the ammonium salt comprising four aliphatic alkyl groups having the total carbon atom of not less than 27 has not more than 40 carbon atoms in total.
(4) The antithrombogenic composition of the above-mentioned (2), wherein the ammonium salt comprising four aliphatic alkyl groups having not less than 27 carbon atoms in total is a trialkylmethylammonium salt.
(5) The antithrombogenic composition of the above-mentioned (2), wherein the ammonium salt comprising four aliphatic alkyl groups having not less than 27 carbon atoms in total is a dialkyldimethylammonium salt.
(6) The antithrombogenic composition of any of the above-mentioned (1) to (5), wherein the ammonium salt comprising four aliphatic alkyl groups having not less than 22 and not more than 26 carbon atoms in total is a dialkyldimethylammonium salt.
(7) A medical device comprising the antithrombogenic composition of any of the above-mentioned (1) to (6), which is applied to at least one part of the contact part with blood.

### DETAILED DESCRIPTION OF THE INVENTION

According to the present invention, the antithrombogenic composition is prepared using heparin or a heparin derivative, which is an anticoagulant compound, and a mixture of plural kinds of organic cationic compounds. As the organic cationic compound, several kinds of ammonium salts are used.

The present invention provides an antithrombogenic composition having superior antithrombogenicity, that has not been achieved by a conventional complex comprising one kind of ammonium salt and heparin, by forming a complex of heparin and plural kinds of ammonium salts having different total number of carbon atoms of the four aliphatic alkyl groups.

According to the present invention, the ammonium salt comprising four aliphatic alkyl groups having not less than 22 and not more than 26 carbon atoms in total is generally contained in an amount of not less than 5% and not more than 80%, preferably not less than 10% and not more than 50%, more preferably not less than 15% and not more than 30%, of the total amount of the ammonium salts.

According to the present invention, examples of the ammonium salt comprising four aliphatic alkyl groups having not less than 22 and not more than 26 carbon atoms in total include didecyldimethylammonium salt, didodecyldimethylammonium salt and the like.

In the present invention, of the ammonium salts used to form an ionic complex with heparin or a heparin derivative, the ammonium salts other than those comprising four aliphatic alkyl groups having not less than 22 and not more than 26 carbon atoms in total comprises four aliphatic alkyl groups having not less than 27, preferably not less than 28, more preferably not less than 30, carbon atoms in total. The upper limit of the total carbon atoms is generally not more than 42, preferably not more than 40, more preferably not more than 38. Examples of such ammonium salt include trialkylmethylammonium salt, dialkyldimethylammonium salt and the like. Examples of trialkylmethylammonium salt include tridodecylmethylammonium salt, tridecylmethylammonium salt, tritetradecylammonium salt and the like. Examples of dialkyldimethylammonium salt include ditetradecyldimethylammonium salt, dihexadecyldimethylammonium salt, dioctadecyldimethylammonium salt and the like.

In the present invention, examples of the heparin derivative include those conventionally used in the pertinent field, such as salts thereof (e.g., heparin sodium, heparin potassium, heparin lithium, heparin calcium and the like, particularly alkaline metal salt), modified heparin such as low molecular heparin and epoxidized heparin, and the like. Examples of preferable low molecular heparin include those having a number average molecular weight of about 5,000.

To prepare the antithrombogenic composition of the present invention, for example, a mixture of the above-mentioned ammonium salts and heparin or a heparin derivative is mixed and stirred in a solvent, such as a mixed solvent of water and organic solvent (e.g., ethanol, methanol, tetrahydrofuran, N,N-dimethylformamide, N,N-dimethylacetamido), to give an ionic derivative as a precipitate. Then, this complex is recovered and washed, and unreacted heparin and ammonium salt are removed. The heparin-ammonium salt complex is dissolved in a solvent. This solution is brought into contact with the surface of a medical device, and the solvent is removed to give an antithrombogenic surface optimized as the surface of a medical device that comes into direct contact with blood.

The above-mentioned solvent, in which a heparin-ammonium salt complex is dissolved, can be any as long as it does not damage the surface of the medical device, which is a base, to the greatest degree possible, and is not particularly limited. Generally, an organic solvent is used, such as methanol, ethanol, isopropyl alcohol, n-propyl alcohol, n-hexane, cyclohexane, tetrahydrofuran, 1,4-dioxane, cyclohexanone, N,N-dimethylformamide, N,N-dimethylacetamide, N-methylpyrrolidone and the like.

A heparin-ammonium salt complex is brought into contact with the surface of a medical device by, for example, coating the surface by immersion, spraying, painting with a brush and the like or other methods. The method is not limited to those exemplified. Another method may comprise dissolving ammonium salts in a suitable solvent in advance, bringing the surface of a medical device into contact with the solution, and after removing the solvent by drying, bringing an aqueous heparin solution into contact therewith to form a heparin-ammonium salt complex on the surface of the medical device.

In the above-mentioned method, the solvent, in which the ammonium salts are dissolved, is not particularly limited as long as it does not damage the surface of a medical device, which is a base, as far as possible. Generally, an organic solvent is used, such as methanol, ethanol, isopropyl alcohol, n-propyl alcohol, n-hexane, cyclohexane, tetrahydrofuran, 1,4-dioxane, cyclohexanone, N,N-dimethylformamide, N,N-dimethylacetamide, N-methylpyrrolidone and the like.

The medical device is not particularly limited as long as it comes into direct contact with blood. Examples thereof include circuit tubes for blood, tubes for sampling monitor, intraaortic balloon pumps, blood pumps for artificial heart, angiography catheters, artificial lung, venous reservoir, artificial kidney, artery filters and the like.

The material of the medical device is not particularly limited as long as it is generally used for a medical device that comes into direct contact with blood. Examples thereof include poly(vinyl chloride), polycarbonate, poly(ethylene terephthalate), polyethylene, polypropylene, polymethylpentene, thermoplastic polyether polyurethane, thermosetting polyurethane, silicone rubber such as polydimethylsiloxane having a crosslinked part and the like, polymethylmethacrylate, poly(vinilidene fluoride), poly(ethylene tetrafluoride), polysulfone, polyethersulfone, polyacetal, polystyrene, ABS resin and a mixture of these resins, metal such as stainless, titanium, aluminum etc., and the like.

### Examples

The present invention is explained in detail by referring to examples. The present invention is not limited by these examples in any way.

### Example 1

Didodecyldimethylammonium bromide (14 parts by weight, manufactured by Tokyo Kasei Kogyo Co., Ltd.) and tridodecylmethylammonium bromide (6 parts by weight, manufactured by Polysciences, Inc.) were dissolved in methanol (200 parts by weight).

Then, heparin (10 parts by weight) was dissolved in ion exchanged water (100 parts by weight), and a solution of the above-mentioned ammonium salts dissolved in methanol was added dropwise with stirring. Since a white precipitate was obtained immediately after the dropwise addition, the liquid was removed by filtration upon completion of the dropwise addition of the solution. Thereafter, unreacted heparin, didodecyldimethylammonium bromide and tridodecylmethylammonium bromide were removed from the precipitate by several repeats of washing with water and methanol. Lyophilization gave a complex (Example 1) of heparin and ammonium salts of the present invention.

### Example 2

Didecyldimethylammonium bromide (2 parts by weight, manufactured by Tokyo Kasei Kogyo Co., Ltd.) and tridodecylmethylammonium bromide (18 parts by weight, manufactured by Polysciences, Inc.) were dissolved in methanol (200 parts by weight).

Then, heparin (10 parts by weight) was dissolved in ion exchanged water (100 parts by weight), and a solution of the above-mentioned ammonium salts dissolved in methanol was added dropwise with stirring. The obtained precipitate was purified by the same method as in Example 1 to give a complex (Example 2) of heparin and ammonium salts of the present invention.

### Example 3

Didodecyldimethylammonium bromide (5 parts by weight, manufactured by Tokyo Kasei Kogyo Co., Ltd.) and distearyldimethylammonium bromide (15 parts by weight, manufactured by Polysciences, Inc.) were dissolved in methanol (200 parts by weight).

Then, heparin (10 parts by weight) was dissolved in ion exchanged water (100 parts by weight), and a solution of the above-mentioned ammonium salts dissolved in methanol was added dropwise with stirring. The obtained precipitate was purified by the same method as in Example 1 to give a complex (Example 3) of heparin and ammonium salts of the present invention.

### Example 4

Didecyldimethylammonium bromide (6 parts by weight, manufactured by Tokyo Kasei Kogyo Co., Ltd.) and dipalmityldimethylammonium bromide (14 parts by weight, manufactured by Polysciences, Inc.) were dissolved in methanol (200 parts by weight).

Then, heparin (10 parts by weight) was dissolved in ion exchanged water (100 parts by weight), and a solution of the above-mentioned ammonium salts dissolved in methanol was added dropwise with stirring. The obtained precipitate was purified by the same method as in Example 1 to give a complex (Example 4) of heparin and ammonium salts of the present invention.

### Comparative example 1

Didodecyldimethylammonium bromide (20 parts by weight, manufactured by Tokyo Kasei Kogyo Co., Ltd.) was dissolved in methanol (200 parts by weight).

Then, heparin (10 parts by weight) was dissolved in ion exchanged water (100 parts by weight), and a solution of the above-mentioned ammonium salt dissolved in methanol was added dropwise with stirring. The obtained precipitate was purified by the same method as in Example 1 to give a complex (Comparative example 1) of heparin and ammonium salt.

### Comparative example 2

Dioctadecyldimethylammonium bromide (20 parts by weight, manufactured by Tokyo Kasei Kogyo Co., Ltd.) was dissolved in methanol (200 parts by weight).

Then, heparin (10 parts by weight) was dissolved in ion exchanged water (100 parts by weight), and a solution of the above-mentioned ammonium salt dissolved in methanol was added dropwise with stirring. The obtained precipitate was purified by the same method as in Example 1 to give a complex (Comparative example 2) of heparin and ammonium salt.

### Comparative example 3

Tridodecylmethylammonium bromide (20 parts by weight, manufactured by Polysciences, Inc.) was dissolved in methanol (200 parts by weight).

Then, heparin (10 parts by weight) was dissolved in ion exchanged water (100 parts by weight), and a solution of the above-mentioned ammonium salt dissolved in methanol was added dropwise with stirring. The obtained precipitate was purified by the same method as in Example 1 to give a complex (Comparative example 3) of heparin and ammonium salt.

### Comparative example 4

Benzyldimethylstearylammonium bromide (20 parts by weight, manufactured by Tokyo Kasei Kogyo Co., Ltd.) was dissolved in methanol (200 parts by weight).

Then, heparin (10 parts by weight) was dissolved in ion exchanged water (100 parts by weight), and a solution of the above-mentioned ammonium salt dissolved in methanol was added dropwise with stirring. The obtained precipitate was purified by the same method as in Example 1 to give a complex (Comparative example 4) of heparin and ammonium salt.

### Evaluation test 1

The complexes obtained in Examples and Comparative examples were each dissolved in THF at a concentration of 0.2% and applied onto a poly(vinyl chloride) tube (PVC tube) having an inner diameter of 3 mm.

The both ends of the above-mentioned tube were cramped into a test tube (length 5 cm). Bovine blood (1.5 ml) added with trisodium citrate for prevention of coagulation was placed in this tube and incubated at 37°C. Thereto was added a 1/40 N calcium chloride solution to initiate coagulation of the blood. After incubation for 3 minutes, the aqueous trisodium citrate solution was again added to stop the coagulation of the blood. The thrombus formed in the tube was collected and weighed. As a control, an uncoated PVC tube was subjected to the same test. The results are shown in Table 1. The values in Table 1 are relative to the weight of the thrombus formed in a glass test tube having the same diameter.

**Table 1**

| | Relative thrombus ratio (glass test tube = 1) |
|---|---|
| Ex. 1 | 0.2 |
| Ex. 2 | 0.2 |
| Ex. 3 | 0.0 |
| Ex. 4 | 0.0 |
| Comparative ex. 1 | 0.1 |
| Comparative ex. 2 | 0.8 |
| Comparative ex. 3 | 0.8 |
| Comparative ex. 4 | 0.1 |
| uncoated PVC | 0.9 |

### Evaluation test 2

For evaluation of the duration of the antithrombogenicity of the composition of the present invention, tubes coated with the complexes obtained in Examples and Comparative examples were immersed in 4N concentrated brine for a week and treated in the same manner as in Evaluation test 1. As a control, an uncoated PVC tube was subjected to the same test. The results are shown in Table 2. The values in Table 2 are relative to the weight of the thrombus formed in a glass test tube having the same diameter.

**Table 2**

| | Relative thrombus ratio (glass test tube = 1) |
|---|---|
| Ex. 1 | 0.3 |
| Ex. 2 | 0.1 |
| Ex. 3 | 0.1 |
| Ex. 4 | 0.1 |
| Comparative ex. 1 | 0.8 |
| Comparative ex. 2 | 0.9 |
| Comparative ex. 3 | 0.9 |
| Comparative ex. 4 | 0.9 |
| uncoated PVC | 0.9 |

### Evaluation test 3

Tubes having an inner diameter of 3 mm and a length of 1 m were coated with a 1% THF solution of the complexes obtained in Examples and Comparative examples and one end thereof was connected to a three way stopcock. Citrated fresh blood taken from rabbits (Japanese White) was passed from one end of the three way stopcock. Simultaneously, a 1/40 N calcium chloride solution was passed from the other end. The calcium chloride solution was injected with a syringe pump at 5 ml/min, and the fresh blood was injected with a syringe pump at 50 ml/min. The blood was reactivated in the tube and started to coagulate. After the complete passage of the blood, the thrombus formed was searched and the distance of the site of the thrombus from the end of the tube was measured. The results are shown in Table 3.

**Table 3**

| | Distance (cm) from end to thrombus |
|---|---|
| Ex. 1 | 13.0 |
| Ex. 2 | 1.0 |
| Ex. 3 | 0.0 |
| Ex. 4 | 0.0 |
| Comparative ex. 1 | 0.0 |
| Comparative ex. 2 | 95.0 |
| Comparative ex. 3 | 96.0 |
| Comparative ex. 4 | 0.0 |
| uncoated PVC | 100.0 |
| Note: 0 means no incidence of thrombus. | |

### Evaluation test 4

For evaluation of the duration of the antithrombogenicity of the composition of the present invention, 4N brine warmed to 37°C was circulated for one week and treated in the same manner as in Evaluation test 3. The results are shown in Table 4.

**Table 4**

| | Distance (cm) from end to thrombus |
|---|---|
| Ex. 1 | 15.0 |
| Ex. 2 | 2.0 |
| Ex. 3 | 0.0 |
| Ex. 4 | 0.0 |
| Comparative ex. 1 | 98.0 |
| Comparative ex. 2 | 97.0 |
| Comparative ex. 3 | 98.0 |
| Comparative ex. 4 | 97.0 |
| uncoated PVC | 100.0 |
| Note: 0 means no incidence of thrombus. | |

### Evaluation test 5

The complexes obtained in Examples and Comparative examples were each dissolved in THF at a concentration of 1% and sprayed onto a poly(vinyl chloride) sheet used for a medical blood bag. This sheet was cut into pieces, placed in a test tube, and the heparin activity on the sheet surface was measured with testzym heparin S (Daiichi Pure Chemicals Co., Ltd.).

To be specific, the sheet was cut into about 5 mm one side squares, and placed in a 24 well microplate for cell culture. While stirring in a thermostat bath at 37°C, an antithrombin III solution and factor Xa were added. Two minutes later, substrate S-2222 was added and, 10 minutes later, acetic acid was added, which was followed by measurement of absorption at 405 nm by a spectrophotometer.

The results are shown in Table 5.

**Table 5**

| | anti-FXa activity (I.U./sq.cm) on the surface |
|---|---|
| Ex. 1 | 1.8 |
| Ex. 2 | 1.9 |
| Ex. 3 | 2.2 |
| Ex. 4 | 2.3 |
| Comparative ex. 1 | 1.8 |
| Comparative ex. 2 | 1.1 |
| Comparative ex. 3 | 1.0 |
| Comparative ex. 4 | 1.6 |

### Evaluation test 6

For evaluation of the duration of the antithrombogenicity, saline was placed in a test tube and incubated for 24 hours. The heparin activity on the sheet surface was measured in the same manner as in Evaluation test 5. The results are shown in Table 6.

**Table 6**

| | anti-FXa activity (I.U./sq.cm) on the surface |
|---|---|
| Ex. 1 | 1.0 |
| Ex. 2 | 1.1 |
| Ex. 3 | 1.2 |
| Ex. 4 | 1.2 |
| Comparative ex. 1 | 0.1 |
| Comparative ex. 2 | 0.7 |
| Comparative ex. 3 | 0.6 |
| Comparative ex. 4 | 0.1 |

As is evident from these evaluation results, the complex of heparin and ammonium salts comprising plural kinds of ammonium salts having specific total numbers of carbon atoms of the present invention has superior antithrombogenicity as compared to a conventional complex comprising one kind of ammonium salt and heparin.

## Claims

1. An antithrombogenic composition comprising an ionic complex of ammonium salts and heparin or a heparin derivative, said ammonium salts each comprising four aliphatic alkyl groups bonded thereto, wherein an ammonium salt comprising four aliphatic alkyl groups having not less than 22 and not more than 26 carbon atoms in total is contained in an amount of not less than 5% and not more than 80% of the total ammonium salt by weight.

2. The antithrombogenic composition of claim 1, wherein an ammonium salt other than the ammonium salt comprising four aliphatic alkyl groups having not less than 22 and not more than 26 carbon atoms in total comprises four aliphatic alkyl groups having not less than 27 carbon atoms in total.

3. The antithrombogenic composition of claim 2, wherein the ammonium salt comprising four aliphatic alkyl groups having not less than 27 carbon atoms in total has not more than 40 carbon atoms in total.

4. The antithrombogenic composition of claim 2, wherein the ammonium salt comprising four aliphatic alkyl groups having not less than 27 carbon atoms in total is a trialkylmethylammonium salt.

5. The antithrombogenic composition of claim 2, wherein the ammonium salt comprising four aliphatic alkyl groups having not less than 27 carbon atoms in total is a dialkyldimethylammonium salt.

6. The antithrombogenic composition of any of claims 1 to 5, wherein the ammonium salt comprising four aliphatic alkyl groups having not less than 22 and not more than 26 carbon atoms in total is a dialkyldimethylammonium salt.

7. A medical device comprising the antithrombogenic composition of any of claims 1 to 6, which is applied to at least one part of the contact part with blood.
